# EUROPÄISCHE PATENTSCHRIFT

(11) **EP 4 009 750 B1**
(45) Veröffentlichungstag und Bekanntmachung des Hinweises auf die Patenterteilung: **15.11.2023**
(21) Anmeldenummer: 21209794.3
(22) Anmeldetag: 23.11.2021
(51) Int. Cl.: H05H 1/24, A61L 2/14, D06M 10/02

(54) **PLASMA-VORRICHTUNG**
PLASMA DEVICE
DISPOSITIF PLASMA

(30) Priorität: 01.12.2020 DE 102020215100
(43) Veröffentlichungstag der Anmeldung: 08.06.2022
(73) Patentinhaber: BSH Hausgeräte GmbH, 81739 München (DE)
(72) Erfinder: Hassfurter, Stefan, 96126 Maroldsweisach (DE); Wende, Tobias, 83209 Prien (DE); Wald, Andreas, 36160 Dipperz (DE)

(56) Entgegenhaltungen:
- WO-A1-2015/110787
- WO-A1-2020/083993
- DE-A1-102018 209 735
- DE-A1-102018 213 143
- DE-B3-102017 118 568

## Beschreibung

Die vorliegende Erfindung betrifft eine Plasma-Vorrichtung zum Behandeln von Oberflächen, insbesondere bei Textilien, gemäß dem Oberbegriff des Anspruchs 1.

Aus der DE 10 2011 100 751 A1 ist eine gattungsgemäße Plasma-Vorrichtung zur Inaktivierung vorzugsweise geruchsrelevanter Moleküle und damit zum Auffrischen von Oberflächen, beispielsweise bei Textilien, bekannt. Die Plasma-Vorrichtung weist ein Gehäuse sowie eine zugeordnete Plasmaquelle auf.

Aus der CN 108 771 767 A ist eine Reinigungsvorrichtung zum Reinigen von Kleidern bekannt, mit einem Gehäuse, in dem ein Aufnahmeraum zur Aufnahme eines Gebläses vorgesehen ist. Ebenfalls in dem Aufnahmeraum vorgesehen ist ein Abscheider, der einen Lufteinlassbereich von einem Reinigungsbereich trennt. Die genannten Bereiche sind dabei im Betriebszustand vertikal übereinander angeordnet. Mit der Reinigungsvorrichtung lassen sich die zu behandelnden Kleidungsstücke insbesondere auch von Zigarettenrauch befreien bzw. reinigen.

Weitere gattungsgemäße Plasmavorrichtungen zur Behandlung von Oberflächen sind aus den Dokumenten WO 2020/083993 A1, DE 10 2018 213143 A1, WO 2015/110787 A1 und DE 10 2017 118568 B3 bekannt.

Zur Reinigung von Kleidungsstücken können diese gewaschen bzw. auch chemisch gereinigt werden, wobei zunehmend auch eine Auffrischung bzw. Behandlung mittels Plasma in den Fokus der Betrachtung rückt, wobei dieses Plasma in der Lage ist, Geruchsmoleküle zu zerstören und dadurch negative Gerüche zu eliminieren. Gleichzeitig wirkt derartiges Plasma antibakteriell und antimikrobiell.

Neben den drei Aggregatzuständen fest, flüssig oder gasförmig, wird Plasma als vierter Aggregatzustand bezeichnet. Wird einem Gas oder Gasgemisch hinreichend viel Energie, beispielsweise in Form von elektrischer Energie, zugefügt, so werden einige Atome des Gases ionisiert, d.h. Elektronen werden aus der Atomhülle entfernt und bewegen sich als freie Teilchen, sodass ein positiv geladenes Atom zurückbleibt. Wenn ein Gas aus einem ausreichend hohen Anteil an freien Ionen und Elektronen besteht, so bezeichnet man den Aggregatzustand als Plasma. Plasma ist also Materie, dessen Bestandteile teilweise geladene Komponenten, Ionen und Elektronen sind, die sich als freie Ladungsträger bewegen.

Nichtthermischem Plasma, das auch als kaltes Plasma bezeichnet wird, kann gezielt zur Beseitigung von Gerüchen und bestimmten Kohlenwasserstoffen eingesetzt werden. Weiterhin finden nichtthermische Plasmen Anwendung in der Medizintechnik, zum Beispiel bei der Behandlung von schlecht oder nicht heilenden Wunden mit Hilfe eines sogenannten Plasmastifts, indem auf die antimikrobielle Wirkung von "kaltem Plasma" zurückgegriffen wird.

Die Ursachen der antibakteriellen Wirkung eines Plasmas liegen in Hitze, Austrocknung, Scherspannung, UV-Strahlung, freie Radikale und Ladungen. Bei kalten Plasmen, wie sie in der erfindungsgemäßen Plasma-Vorrichtung verwendet werden, spielt die Hitze eine untergeordnete Rolle, da diese Plasmen bei Raumtemperatur betrieben werden. In solchen Niederdruckplasmen entstehen besonders reaktive Partikel, wie beispielsweise verschiedene Sauerstoff- oder Stickstoffspezies, die eine ausreichend hohe Lebensdauer aufweisen, um bei einer indirekten Exposition organische Verbindungen zu schädigen. Zu diesen Partikeln zählen unter anderem atomarer Sauerstoff, Superoxidradikale, Ozon, Hydroxylradikale, Stickstoffmonooxid und Stickstoffdioxid. Diese Partikel zeigen eine zerstörerische Wirkung auf unterschiedlichste Geruchskomponenten wie auch Zellkomponenten. Werden Geruchskomponenten, welche meist aus Kohlenstoffverbindungen bestehen, wie auch Zellwände von Bakterien, Keimen, Viren, Pilzen oder anderen vergleichbaren Mikroorganismen dem Plasma direkt ausgesetzt, so laden sich diese aufgrund des Beschusses mit den im Plasma vorhandenen Elektronen negativ auf. Aufgrund der elektrostatischen Abstoßung führt dies zu mechanischen Spannungen bis hin zur Überschreitung der Zugfestigkeit und der Zerstörung von dem Geruchsmolekül, bzw. der Zellwand. Aber nicht nur mechanische Verspannungen aufgrund der Ladung können die Zellwände zerstören, sondern auch die Störung des Ladungsgleichgewichts der Geruchsmoleküle, bzw. der Zellwand durch verschiedene, weitere elektrostatische Wechselwirkungen und der Elektrolyse, z. B. durch Änderung der Permeabilität der Zellwände. Ein Mechanismus zur Inaktivierung von Mikroorganismen ergibt sich auch aus den sehr energiereichen Ionen. Mittels einer Hochfrequenz kann das Plasma erzeugt werden. Niederdruckplasmen sind daher besonders gut zur Inaktivierung von Gerüchen an textilem Gewebe oder von haushaltsüblichen Oberflächen oder dergleichen geeignet, um eine Geruchsaktivierung zu erreichen.

Die zu behandelnden Oberflächen können dabei textile Materialien mit natürlichen, pflanzlichen oder tierischen Fasern, wie z. B. Baum-, Schafwolle, Seide, Leinen, Filz oder künstlichen Fasern, wie z. B. Nylon, sein. Weiter sollen unter dem Begriff "Oberfläche" auch Materialien und Gegenstände aus Keramik, Kunststoff, Federn, Leder, Glas, Holz, oder Metall verstanden werden können.

Nachteilig bei der aus dem Stand der Technik bekannten Plasma-Vorrichtung ist, dass ein Energiespeicher so eingebaut ist, dass selbst bei einem geöffneten Gehäuse eine Spannungsversorgung unter Umständen nicht unterbrochen ist. Dies könnte gegebenenfalls jedoch zu Schäden an einer Elektronikeinheit bzw. der Plasmaquelle führen.

Die vorliegende Erfindung beschäftigt sich daher mit dem Problem, für eine Plasma-Vorrichtung der gattungsgemäßen Art eine verbesserte oder zumindest eine alternative Ausführungsform anzugeben, die insbesondere elektronische Komponenten der Plasma-Vorrichtung bei einer Wartung bzw. einer Reparatur oder auch bei einem Sturz vor unerwünschter elektrischer Spannung schützt.

Dieses Problem wird erfindungsgemäß durch den Gegenstand des unabhängigen Anspruchs 1 gelöst. Vorteilhafte Ausführungsformen sind Gegenstand der abhängigen Ansprüche.

Die vorliegende Erfindung beruht auf dem allgemeinen Gedanken, eine an sich bekannte Plasma-Vorrichtung zum Behandeln von Oberflächen, insbesondere zum Auffrischen von Textilien, mit einem Gehäuse mit zwei Schalen, nämlich einer Oberschale und einer Unterschale auszustatten und eine Spannungsversorgung einer Elektronikeinheit bzw. einer Plasmaquelle der Plasma-Vorrichtung innerhalb des Gehäuses konstruktiv so auszulegen, dass diese unterbrochen wird, sofern das Gehäuse über ein bestimmtes Maß hinaus geöffnet wird. Die erfindungsgemäße Plasma-Vorrichtung weist hierzu ein Gehäuse mit einer Unterschale sowie einer schwenkbar daran gelagerten Oberschale auf, wobei in der Oberschale eine Aufnahme für den elektrischen Energiespeicher, beispielsweise eine Batterie oder einen Akkumulator, vorgesehen ist. In der Unterschale sind die Plasmaquelle sowie die Elektronikeinrichtung, beispielsweise eine Leiterplatine, angeordnet, wobei die Elektronikeinrichtung elektrische Kontaktelemente aufweist, die bei geschlossenem Gehäuse, das heißt bei fest mit der Unterschale verbundener Oberschale elektrisch leitend mit dem Energiespeicher verbunden sind und dadurch den Energiespeicher elektrisch leitend mit der Elektronikeinrichtung und darüber mit der Plasmaquelle verbinden. Ab einem Öffnungswinkel α der Oberschale relativ zur Unterschale von α ≥ 5° ist die Spannungsversorgung erfindungsgemäß unterbrochen, so dass der Energiespeicher ab dem zuvor genannten Öffnungswinkel α nicht mehr elektrisch leitend mit den Kontaktelementen verbunden ist. Hierdurch kann erreicht werden, dass die Elektronikeinrichtung, beispielsweise eine Platine, vor Spannungsschäden geschützt ist, die beispielsweise durch einen ungewollt fließenden Strom bzw. eine unerwünschte Spannung in der Platine gegeben sein können. Zudem kann mit der Variante mit Oberschale und Unterschale eine zuverlässige und kostengünstige Variante geschaffen werden, sowohl die Elektronikeinrichtung als auch die Plasmaquelle spannungsfrei zu bekommen, ohne dass hierfür weitere Bauteile, wie beispielsweise Schrauben, Klappen, Schnapphaken oder ähnliches erforderlich wären. Die an die Schließstellung des Gehäuses der Plasma-Vorrichtung gekoppelte Spannungsversorgung der Elektronikeinrichtung und der Plasmaquelle bieten darüber hinaus den großen Vorteil, dass bei einem Herunterfallen der Plasma-Vorrichtung und einem Öffnen des Gehäuses der elektrische Energiespeicher unmittelbar elektrisch von den Kontaktelementen der Elektronikeinrichtung getrennt wird. Dabei ist das Gehäuse üblicherweise so konstruiert, dass sich dieses bei einem Sturz öffnen kann und dadurch die Spannungsversorgung der Elektronikeinrichtung bzw. der Plasmaquelle unterbrochen wird. Der Öffnungswinkel α erstreckt sich dabei vorzugsweise in einer vertikalen Längsebene.

Bei einer vorteilhaften Weiterbildung der erfindungsgemäßen Lösung sind die Oberschale und die Unterschale in geschlossenem Zustand über ein Rastelement und/oder eine Schraube und/oder einem Splint aneinander fixiert. Selbstverständlich ist alternativ auch eine unter bestimmten Belastungen aufgehende Verklebung zwischen der Oberschale und der Unterschale denkbar, wobei insbesondere ein Rastelement ein einfaches Schließen des Gehäuses durch beispielsweise ein Aneinanderdrücken der Oberschale an die Unterschale ohne weiteres Werkzeug ermöglicht. Das Öffnen des Gehäuses kann durch ein Zurückdrücken des Rastelements und damit einem Lösen seiner Raststellung erreicht werden. Alternativ ist selbstverständlich auch denkbar, dass die Oberschale und die Unterschale im geschlossenen Zustand über eine Schraube aneinander fixiert sind, wobei eine derartige Schraube beispielsweise über einen entsprechenden Kopf mit einem entsprechenden Werkzeugeingriff eine Art Schlossfunktion darstellen kann, so dass die Schraube nur mit einem dazu passend ausgebildeten Werkzeug (Schlüssel) aufgedreht und das Gehäuse geöffnet werden kann. Hierdurch kann insbesondere die Gefahr eines Öffnens des Gehäuses durch Unbefugte, beispielsweise Laien, größtenteils ausgeschlossen werden. Wiederum alternativ kann auch ein Splint vorgesehen sein, der ein einfaches und nutzerfreundliches Öffnen ermöglicht.

Zweckmäßig beträgt bei einem Öffnungswinkel α der Oberschale relativ zur Unterschale von ca. 5° ein maximaler Abstand d der Oberschale von der Unterschale ca. 14 mm. Der Abstand d ist dabei an einem der Schwenkachse gegenüber liegenden Längsende der Plasma-Vorrichtung abgenommen. Mit einem derartigen Abstand ist ein geringfügiges Aufschwenken der Oberschale relativ zur Unterschale möglich, ohne dass dabei die Spannungsversorgung der Elektronikeinrichtung bzw. der Plasmaquelle unterbrochen wird, während ein weiteres Öffnen des Gehäuses, das heißt ein weiteres Aufschwenken der Oberschale zwangsläufig zu einem Kontaktverlust zwischen dem Energiespeicher und den Kontaktelementen und damit einem stromlos schalten der Elektronikeinrichtung bzw. der Plasmaquelle führt. Rein theoretisch ist dabei denkbar, dass bei einem weiteren Öffnen der Oberschale über einen Winkel von α = 5° weitere Rastelemente gelöst werden müssen, so dass bei einem einfachen Herunterfallen der Plasma-Vorrichtung auf einen Fußboden zwar ein Öffnen des Gehäuses erfolgt, dies jedoch noch nicht unbedingt zu einem elektrischen Entkoppeln des Energiespeichers von der Elektronikeinrichtung führt. Dies bietet den großen Vorteil, dass die Plasma-Vorrichtung wieder aufgehoben und durch ein Zudrücken der Oberschale eine Wiederinbetriebnahme möglich ist. Für Reparatur- und Wartungszwecke genügt der Öffnungswinkel α von 5° nicht aus, so dass für diese Zwecke die Oberschale weiter aufgeschwenkt und damit das Gehäuse weiter geöffnet werden muss. Dies wiederum könnte beispielsweise nur dann möglich sein, sofern weitere Rastelemente gelöst werden.

Bei einer vorteilhaften Weiterbildung der erfindungsgemäßen Lösung sind die Kontaktelemente an der Elektronikeinrichtung als Federbügel ausgebildet. Derartige Federbügel ermöglichen eine Federvorspannung der Kontaktelemente gegen die Pole des Energiespeichers, wodurch eine zuverlässige Energieübertragung ermöglicht wird.

Durch die Federbügel können zudem Maßtoleranzen bzw. Fertigungstoleranzen kompensiert werden.

Zweckmäßig sind an der Oberschale ein baggerschaufelartiges Element und an der Unterschale eine komplementär dazu ausgebildete Führung angeordnet, in der das Element bei einer Schwenkbewegung der Oberschale geführt ist. Ein derartiges baggerschaufelartiges Element sowie eine komplementär dazu ausgebildete Führung ermöglichen eine Schwenkbewegung der Oberschale relativ zur Unterschale und zugleich ein Abnehmen der Oberschale von der Unterschale, sofern ein vordefinierter Öffnungswinkel, beispielsweise α > 90° überschritten wird. Im Vergleich zu beispielsweise einem Scharnier lässt sich eine derartige Element-Führung-Verbindung konstruktiv einfach realisieren. Eine solche Verbindung erleichtert auch eine Reparatur der Plasma-Vorrichtung, da für Reparatur- und Wartungszwecke die Oberschale ganz von der Unterschale abgenommen werden kann und dadurch Reparaturarbeiten nicht mehr behindert, ohne dass beispielsweise ein Scharnier erst von der Oberschale oder der Unterschale abgeschraubt werden müsste.

Zweckmäßig ist an der Unterschale eine in Längsrichtung des Gehäuses verlaufende Plateaufläche mit zumindest einer Elektrode vorgesehen, über die Plasma erzeugbar bzw. auf die zu behandelnde Oberfläche ausbringbar ist, wobei die Plateaufläche federbeaufschlagt ist und in einem Nichtgebrauchszustand der Plasma-Vorrichtung über das Gehäuse übersteht. Die Elektrode kann ebenfalls leicht in Bezug auf die Plateaufläche erhaben ausgebildet sein und beispielweise eine linienartige Gestalt aufweisen. Die Elektrode kann dabei von einem Keramiksubstrat umgeben sein. Durch eine Federbeaufschlagung der Plateaufläche ist es möglich, diese beim Reinigungsvorgang dicht auf der Oberfläche zu führen, wodurch insbesondere eine übermäßige Ozonproduktion durch die Plasmaquelle verhindert werden kann. Die federbeaufschlagte Plateaufläche bewirkt auch ein Abschirmen des ausgegebenen Plasmas vom Luftsauerstoff, so dass zwar genügend Sauerstoff zur Ozonproduktion zur Verfügung steht, die zuvor genannte übermäßige Ozonproduktion mit den damit verbundenen Nachteilen jedoch nicht stattfindet. Die Nachteile einer übermäßigen Ozonproduktion liegen insbesondere bei einer längeren Einwirkung auf die zu behandelnde Oberfläche, beispielsweise eine Textilie, in einer Ver- bzw. Entfärbung derselben sowie in der Gefahr von Atemwegsreizungen.

Die mit der erfindungsgemäßen Plasma-Vorrichtung neutralisierbare geruchsrelevanten Moleküle können Buttersäure, Schweiß, festgesetzter Zigarettenrauch oder generell Gerüche, die als unangenehm empfundenen werden können, sein. Die Plasma-Vorrichtung beruht darauf, dass die Gerüche aktiv beseitigt werden und nicht unangenehme Gerüche, z. B. mittels Parfüm, überdeckt werden. Mit der erfindungsgemäßen Plasma-Vorrichtung können auch nicht-geruchsrelevante Moleküle, wie beispielsweise Allergene, Proteinmoleküle, Prionen, u. ä, zerstört werden.

Weitere wichtige Merkmale und Vorteile der Erfindung ergeben sich aus den Unteransprüchen, aus den Zeichnungen und aus der zugehörigen Figurenbeschreibung anhand der Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen sofern sie unter den Schutzumfang der Ansprüche, die die Erfindung definieren, fallen.

Bevorzugte Ausführungsbeispiele der Erfindung sind in den Zeichnungen dargestellt und werden in der nachfolgenden Beschreibung näher erläutert, wobei sich gleiche Bezugszeichen auf gleiche oder ähnliche oder funktional gleiche Komponenten beziehen. Dabei zeigen, jeweils schematisch,
- Fig. 1: eine Ansicht von schräg oben auf die erfindungsgemäße Plasma-Vorrichtung,
- Fig. 2: eine Ansicht von schräg unten auf die erfindungsgemäße Plasma-Vorrichtung,
- Fig. 3: eine Schnittdarstellung durch eine erfindungsgemäße Plasma-Vorrichtung bei geschlossenem Gehäuse,
- Fig. 4: eine Darstellung wie in Fig. 3, jedoch bei einem geöffneten Gehäuse, bei welchem die Oberschale um einen Winkel α von 5° zur Unterschale aufgeschwenkt ist,
- Fig. 5: eine Darstellung wie in Fig. 4, jedoch bei weiter aufgeschwenkter Oberschale,
- Fig. 6: eine Detaildarstellung aus Fig. 3 im Bereich einer Schwenkachse,
- Fig. 7: eine Detaildarstellung aus Fig. 5,
- Fig. 8: eine Ansicht von innen auf die Oberschale,
- Fig. 9: eine Ansicht von innen auf die Unterschale.

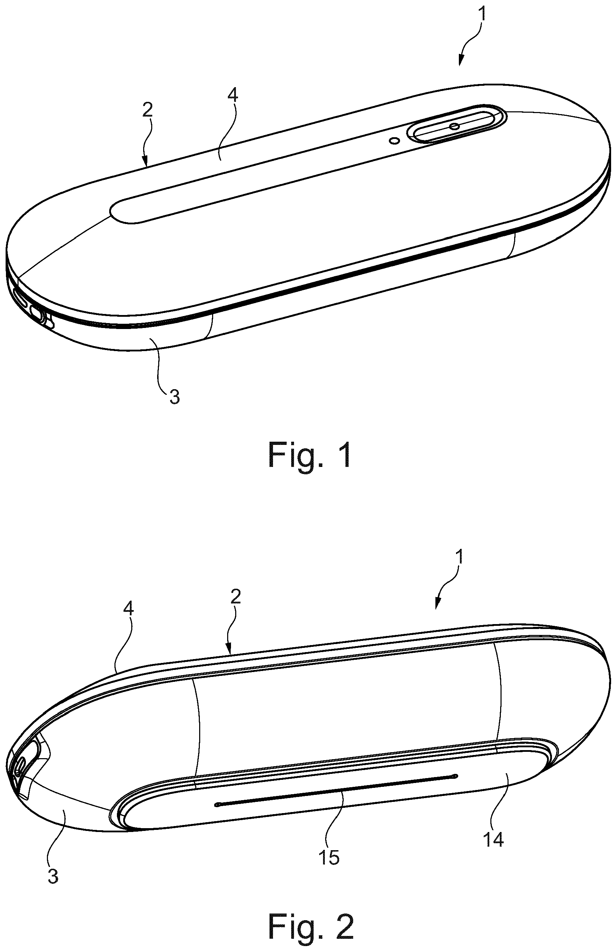

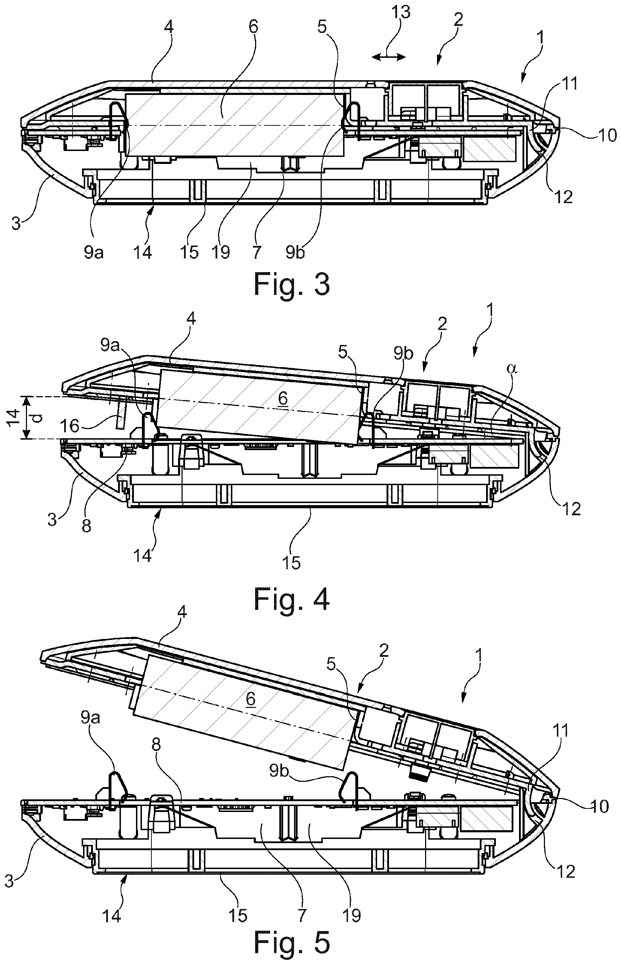

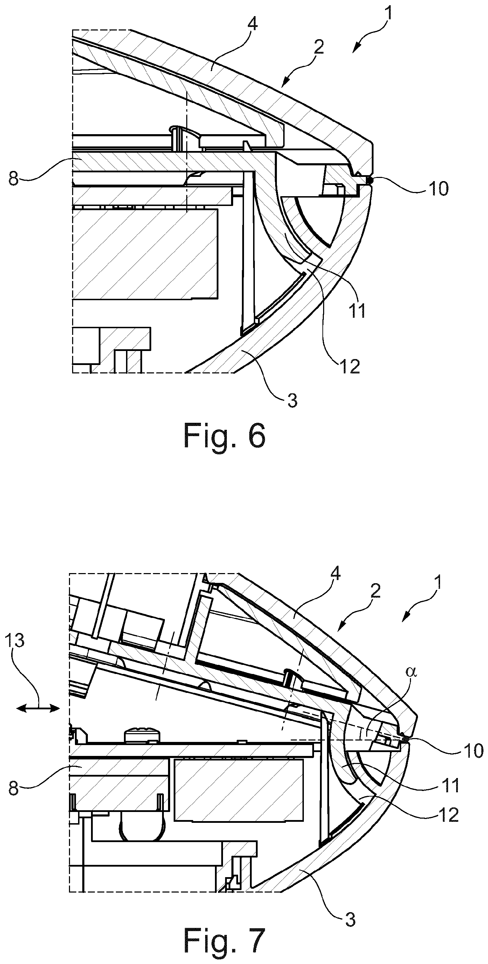

Entsprechend den Fig. 1 bis 7, weist eine erfindungsgemäße Plasma-Vorrichtung 1 zum Behandeln von Oberflächen, beispielsweise zum Auffrischen von Textilien, ein Gehäuse 2 mit einer Unterschale 3 sowie einer schwenkbar daran gelagerten Oberschale 4 auf. In der Oberschale 4 ist dabei eine Aufnahme 5 für einen elektrischen Energiespeicher 6, beispielsweise einen Akkumulator oder eine Batterie, vorgesehen, während in der Unterschale 3 eine Plasmaquelle 7 sowie eine Elektronikeinrichtung 8 mit elektrischen Kontaktelementen 9a und 9b angeordnet ist. Die Elektronikeinrichtung 8 kann dabei beispielsweise als Platine ausgebildet sein und zur Steuerung der Plasmaquelle 7 dienen. Betrachtet man nun die Plasma-Vorrichtung 1 entsprechend der Fig. 3, so kann man erkennen, dass bei einem geschlossenen Gehäuse 2, das heißt bei direkt auf der Unterschale 3 aufliegender Oberschale 4, die elektrischen Kontaktelemente 9a, 9b mit entsprechenden Polen des elektrischen Energiespeichers 6 verbunden sind und dadurch die Elektronikeinrichtung 8 bzw. die Plasmaquelle 7 elektrisch mit dem Energiespeicher 6 verbunden sind.

Wird nun das Gehäuse 2 geöffnet, indem die Oberschale 4 relativ zur Unterschale 3 um eine Schwenkachse 10 aufgeschwenkt wird, so verliert das elektrische Kontaktelement 9a der Elektronikeinrichtung 8 ab einem vordefinierten Öffnungswinkel α ≥ 5° den Kontakt zum elektrischen Energiespeicher 6, wodurch die Spannungsversorgung der Elektronikeinrichtung 8 und damit auch der Plasmaquelle 7 unterbrochen wird. Diese Situation ist gemäß der Fig. 4 dargestellt. Gemäß der Fig. 4 ist genau der Öffnungszustand des Gehäuses 2 gezeigt, bei dem das Kontaktelement 9a gerade noch mit dem Energiespeicher 6 in Verbindung steht. Bei einem Öffnungswinkel α von ca. 5° beträgt ein maximaler Abstand d der Oberschale 4 von der Unterschale 3 ca. 14 mm. Der maximale Abstand d wird dabei auf der der Schwenkachse 10 gegenüberliegenden Seite der Plasma-Vorrichtung 1 abgelesen. Der Öffnungswinkel α liegt dabei in der Blattebene, das heißt in einer vertikalen Längsebene bezogen auf die Plasma-Vorrichtung 1.

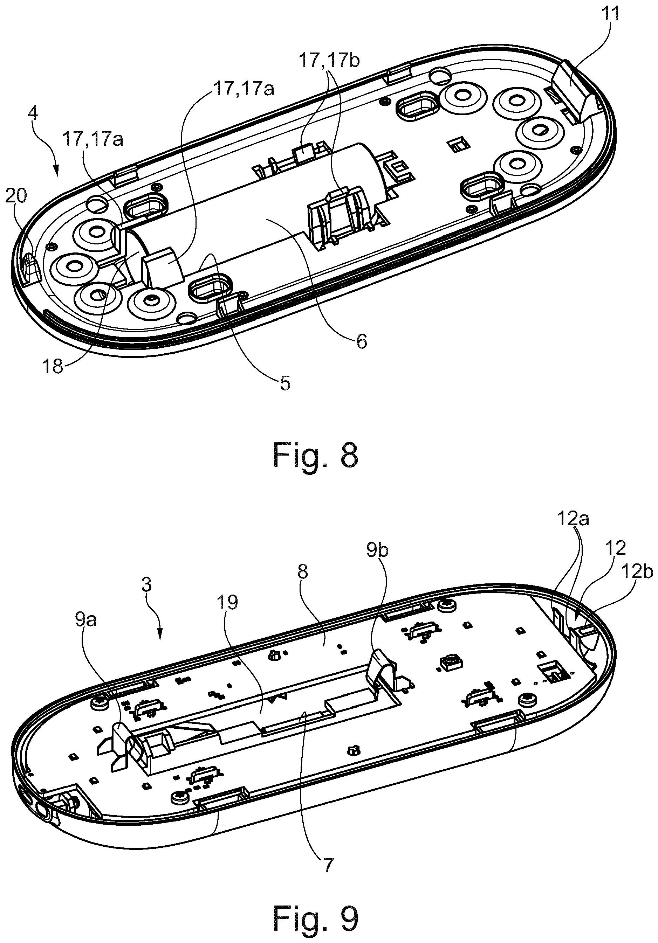

Wird die Oberschale 4 weiter aufgeschwenkt, wie dies gemäß der Fig. 5 gezeigt ist, so verliert auch das Kontaktelement 9b den Kontakt zum elektrischen Energiespeicher 6, wodurch dieser elektrisch komplett isoliert von den Kontaktelementen 9a und 9b ist. Der elektrische Energiespeicher 6 ist dabei in der Aufnahme 5 der Oberschale 4 fixiert, so dass durch ein komplettes Aufklappen der Oberschale 4 der elektrische Energiespeicher 6 einfach von oben in die Aufnahme 5 eingedrückt bzw. wieder aus dieser herausgezogen werden kann. Über entsprechende Halteelemente 17, beispielsweise Klemmelemente, wird der elektrische Energiespeicher 6 auch bei nach unten offener Aufnahme 5, wie diese gemäß den Fig. 3 bis 5 und Fig. 8 dargestellt ist, zuverlässig gehalten. Betrachtet man die Fig. 8 genauer, so kann man erkennen, dass hier an einem Längsende der Aufnahme 5 Halteelemente 17, 17a vorgesehen sind, die den elektrischen Energiespeicher 6 sowohl an seinem Umfang umfassen als auch an seiner Stirnseite 18. Die Halteelemente 17a bilden somit eine Art Schuh für den Energiespeicher 6. Die anderen Halteelemente 17 sind als federnde Klemmelemente 17b ausgebildet und spannen den Energiespeicher 6 in die Aufnahme 5 vor.

Bei geschlossenem Gehäuse 2 greift der elektrische Energiespeicher 6 in eine entsprechende Ausnehmung 19 der Elektronikeinrichtung 8, beispielsweise der Platine, ein. In Fig. 4 ist zusätzlich eine optionale Schutzwand 16 eingezeichnet, die den Energiespeicher 6 schützt und beispielsweise durch Eingreifen in eine zugehörige Öffnung an der Elektronikeinrichtung 8 eine Aussteifung bewirkt. Weiter ist eine direkte Berührung der Kontaktfläche des Energiespeichers 6 mit nadelartigen Gegenständen von der Öffnungsseite her erschwert.

Mit der erfindungsgemäßen Plasma-Vorrichtung 1 kann sichergestellt werden, dass bei einem Öffnen des Gehäuses 2 eine zuverlässige Spannungsunterbrechung an der Elektronikeinrichtung 8 und der Plasmaquelle 7 gewährleistet werden kann, wodurch insbesondere Spannungs- oder Stromschäden zuverlässig vermieden werden können. Auch kann mit dem erfindungsgemäßen Gehäuse 2 vermieden werden, dass bei einem unbeabsichtigten Herabfallen der Plasma-Vorrichtung 1 und Öffnen des Gehäuses 2 eine weitere Spannungs-/Stromversorgung der Elektronikeinrichtung 8 erfolgt.

Die Kontaktelemente 9a, 9b können dabei beispielsweise als Federbügel ausgebildet sein und sind dadurch in der Lage, zumindest geringe Einbautoleranzen des Energiespeichers 6 in der Aufnahme 5, ebenso wie Fertigungstoleranzen problemlos zu kompensieren.

In geschlossenem Zustand des Gehäuses 2 sind die Oberschale 4 und die Unterschale 3 über beispielsweise ein Rastelement 20 (vgl. Fig. 8) oder eine nicht gezeigte Schraube und/oder einen Splint aneinander fixiert, wobei insbesondere das erwähnte Rastelement 20 ein Schließen des Gehäuses 2 ohne weitere Werkzeuge ermöglicht. Durch eine Schraube mit einem entsprechenden Werkzeugeingriff könnte hingegen eine Art Schloss geschaffen werden, welches ein Öffnen der Schraube und damit auch ein Öffnen des Gehäuses 2 ausschließlich mit einem dazu passenden Werkzeug, das heißt einem dazu passenden Schlüssel, ermöglicht.

Betrachtet man die Scharnierfunktion der Oberschale 4 an der Unterschale 3, so kann man erkennen, dass an der Oberschale 4 ein baggerschaufelartiges Element 11 und an der Unterschale 3 eine komplementär dazu ausgebildete Führung 12 angeordnet sind, in der das Element 11 bei einer Schwenkbewegung der Oberschale 4 geführt ist (vgl. auch die Fig. 8 und 9). Durch das Zusammenwirken des Elements 11 mit der Führung 12 kann ein zuverlässiges Aufschwenken der Oberschale 4 relativ zur Unterschale 3 erreicht werden. Im Unterschied zu einem Scharnier, bei welchem die Oberschale 4 drehbar aber ansonsten fest mit der Unterschale 3 verbunden ist, ermöglicht die Lösung mit dem Element 11 und der Führung 12 ein weites Aufklappen der Oberschale 4 sowie auch ein Abnehmen derselben von der Unterschale 3. Die Führung 12 besitzt dabei vorzugsweise zwei kreissegmentartige äußere Führungskonturen 12a sowie eine dazwischen angeordnete innere Führungskontur 12b, über welche das Element 11 sowohl an einer Außenseite als auch an einer Innenseite geführt ist.

In der Oberschale 4 des Gehäuses 2 sind dabei vorzugsweise keine Elektronikkomponenten verbaut, so dass diese lediglich als oberer Abschluss des Gehäuses 2 und zur Aufnahme des elektrischen Energiespeichers 6 dient.

Um darüber hinaus eine möglichst hohe Reinigungsleistung der Plasma-Vorrichtung 1 zu erzielen, ist an der Unterschale 3 eine in Längsrichtung 13 des Gehäuses 2 verlaufende Plateaufläche 14 mit zumindest einer Elektrode 15 vorgesehen, über die Plasma erzeugbar bzw. auf die zu behandelnde Oberfläche ausbringbar ist (vgl. Fig. 2 bis 5). Die Plateaufläche 14 ist dabei vorzugsweise federbeaufschlagt und steht in einem Nichtgebrauchszustand der Plasma-Vorrichtung 1 (vgl. die Fig. 2 bis 5) über das Gehäuse 2, das heißt in diesem Fall über die Unterschale 3 nach unten über. Durch die Federbeaufschlagung der Plateaufläche 14 ist es möglich, diese während der Nutzung der Plasma-Vorrichtung 1 dicht auf der zu reinigenden bzw. aufzufrischenden Oberfläche entlang zu fahren, wodurch insbesondere eine übermäßige Ozonproduktion durch die Plasmaquelle 7 verhindert werden kann. Hierdurch lassen sich sowohl Atemwegsreizungen als auch unerwünschte Ver- bzw. Entfärbungen der zu reinigenden Oberfläche durch eine zu starke Ozonkonzentration verhindern.

Mit der erfindungsgemäßen Plasma-Vorrichtung 1 ist es möglich, eine leichte Zugänglichkeit für Wartungs- und Reparaturzwecke zu schaffen und zugleich gewährleisten, dass die Plasma-Vorrichtung 1 ab einem vordefinierten Öffnungsgrad des Gehäuses 2 vom elektrischen Energiespeicher 6 isoliert und damit stromlos geschalten ist.

### Bezugszeichenliste

- 1: Plasma-Vorrichtung
- 2: Gehäuse
- 3: Unterschale
- 4: Oberschale
- 5: Aufnahme
- 6: Energiespeicher
- 7: Plasmaquelle
- 8: Elektronikeinrichtung
- 9: Kontaktelemente
- 10: Schwenkachse
- 11: Element
- 12: Führung
- 13: Längsrichtung
- 14: Plateaufläche
- 15: Elektrode
- 16: Schutzwand
- 17: Halteelement
- 18: Stirnseite des Energiespeichers
- 19: Ausnehmung
- 20: Rastelement

## Patentansprüche

1. Plasma-Vorrichtung (1) zum Behandeln von Oberflächen, insbesondere bei Textilien, wobei die Plasma-Vorrichtung (1) ein Gehäuse (2) mit einer Unterschale (3) und einer schwenkbar daran gelagerten Oberschale (4) aufweist, wobei in der Oberschale (4) eine Aufnahme (5) für einen elektrischen Energiespeicher (6) vorgesehen ist und wobei in der Unterschale (3) eine Plasmaquelle (7) sowie eine damit verbundene Elektronikeinrichtung (8) mit elektrischen Kontaktelementen (9a, 9b) angeordnet sind, welche bei geschlossenem Gehäuse (2) elektrisch leitend mit dem Energiespeicher (6) verbunden sind und dadurch diesen elektrisch leitend mit der Elektronikeinrichtung (8) verbinden, **gekennzeichnet dadurch, dass** ab einem Öffnungswinkel α der Oberschale (4) relativ zur Unterschale (3) von α ≥ 5° der Energiespeicher (6) zumindest von einem Kontaktelement (9a, 9b) entkoppelt ist.

2. Plasma-Vorrichtung nach Anspruch 1, wobei die Oberschale (4) und die Unterschale (3) in geschlossenem Zustand über ein Rastelement (20) und/oder eine Schraube und/oder einem Splint aneinander fixiert sind.

3. Plasma-Vorrichtung nach Anspruch 1 oder 2, wobei bei einem Öffnungswinkel α der Oberschale (4) relativ zur Unterschale (3) von α ≥ 5° ein maximaler Abstand d der Oberschale (4) von der Unterschale (3) ca. 14 mm beträgt.

4. Plasma-Vorrichtung nach einem der Ansprüche 1 bis 3, wobei die Kontaktelemente (9a, 9b) als Federbügel ausgebildet sind.

5. Plasma-Vorrichtung nach einem der Ansprüche 1 bis 4, wobei an der Oberschale (4) ein baggerschaufelartiges Element (11) und an der Unterschale (3) eine komplementär dazu ausgebildete Führung (12) angeordnet sind, in der das Element (11) bei einer Schwenkbewegung der Oberschale (4) geführt ist.

6. Plasma-Vorrichtung nach einem der Ansprüche 1 bis 5, wobei die Oberschale (4) von der Unterschale (3) abnehmbar ist.

7. Plasma-Vorrichtung nach einem der vorhergehenden Ansprüche, wobei an der Unterschale (3) eine in Längsrichtung (13) des Gehäuses (2) verlaufende Plateaufläche (14) mit zumindest einer Elektrode (15) vorgesehen ist, über die Plasma auf die zu behandelnde Oberfläche ausbringbar ist, wobei die Plateaufläche (14) federbeaufschlagt ist und in einem Nichtgebrauchszustand der Plasma-Vorrichtung (1) über das Gehäuse (2) übersteht.

8. Plasma-Vorrichtung nach einem der vorhergehenden Ansprüche, wobei der Energiespeicher (6) eine Batterie oder ein Akkumulator ist.

9. Plasma-Vorrichtung nach einem der vorhergehenden Ansprüche, wobei der Öffnungswinkel α in einer vertikalen Längsebene liegt.

10. Plasma-Vorrichtung nach einem der vorhergehenden Ansprüche, wobei an der Oberschale (4) eine Schutzwand angeordnet ist, die in Richtung der Unterschale (3) absteht.

## Claims

1. Plasma device (1) for treating surfaces, in particular in the case of textiles, wherein the plasma device (1) has a housing (2) with a lower shell (3) and an upper shell (4) pivotably mounted thereon, wherein a seat (5) for an electrical energy storage system (6) is provided in the upper shell (4) and wherein a plasma source (7) and an electronic device (8) connected thereto with electrical contact elements (9a, 9b) are arranged in the lower shell (3), which when the housing (2) is closed are electrically conductively connected to the energy storage system (6) and as a result connect this electrically conductively to the electronic device (8), **characterised in that** as from an angle of opening α of the upper shell (4) relative to the lower shell (3) of α ≥ 5° the energy storage system (6) is decoupled at least from one contact element (9a, 9b).

2. Plasma device according to claim 1, wherein in the closed state the upper shell (4) and the lower shell (3) are fixed to one another by a latching element (20) and/or a screw and/or a splint.

3. Plasma device according to claim 1 or 2, wherein at an angle of opening α of the upper shell (4) relative to the lower shell (3) of α ≥ 5° a maximum distance d of the upper shell (4) from the lower shell (3) is approx. 14 mm.

4. Plasma device according to one of claims 1 to 3, wherein the contact elements (9a, 9b) are designed as spring clips.

5. Plasma device according to one of claims 1 to 4, wherein an element (11) similar to an excavator shovel is arranged on the upper shell (4), and arranged on the lower shell (3) is a guide (12) designed to be complementary thereto, in which the element (11) is guided during a pivoting motion of the upper shell (4).

6. Plasma device according to one of claims 1 to 5, wherein the upper shell (4) can be removed from the lower shell (3).

7. Plasma device according to one of the preceding claims, wherein a plateau area (14) running in the longitudinal direction (13) of the housing (2) and containing at least one electrode (15) is provided on the lower shell (3), by way of which plasma can be applied to the surface to be treated, wherein the plateau area (14) is spring-loaded and when the plasma device (1) is not in use protrudes over the housing (2).

8. Plasma device according to one of the preceding claims, wherein the energy storage system (6) is a battery or an accumulator.

9. Plasma device according to one of the preceding claims, wherein the angle of opening α lies in a vertical longitudinal plane.

10. Plasma device according to one of the preceding claims, wherein a protective wall is arranged on the upper shell (4), and sticks out in the direction of the lower shell (3).

## Revendications

1. Dispositif à plasma (1) pour traiter des surfaces, en particulier des surfaces de textiles, dans lequel le dispositif à plasma (1) présente un boîtier (2) comprenant une coque inférieure (3) et une coque supérieure (4) montée sur celle-ci de manière pivotante,
dans lequel un logement (5) est agencé dans la coque supérieure (4) pour un réservoir d'énergie électrique (6) et
une source de plasma (7) ainsi qu'un dispositif électronique (8) relié à celle-ci avec des éléments de contact électriques (9a, 9b) sont disposés dans la coque inférieure (3), lesquels sont reliés de manière électriquement conductrice au réservoir d'énergie (6) lorsque le boîtier (2) est fermé et relient ainsi celui-ci électriquement au dispositif électronique (8),
**caractérisé en ce qu'**à partir d'un angle d'ouverture α de la coque supérieure (4) par rapport à la coque inférieure (3) de α ≥ 5°, le réservoir d'énergie (6) est découplé au moins d'un élément de contact (9a, 9b).

2. Dispositif à plasma selon la revendication 1, dans lequel la coque supérieure (4) et la coque inférieure (3) sont fixées l'une à l'autre à l'état fermé par le biais d'un élément d'encliquetage (20) et/ou d'une vis et/ou d'une goupille.

3. Dispositif à plasma selon la revendication 1 ou 2, dans lequel, à un angle d'ouverture α de la coque supérieure (4) par rapport à la coque inférieure (3) de α ≥ 5°, une distance maximale d entre la coque supérieure (4) et la coque inférieure (3) est d'environ 14 mm.

4. Dispositif à plasma selon l'une des revendications 1 à 3, dans lequel les éléments de contact (9a, 9b) sont réalisés sous forme d'étriers à ressort.

5. Dispositif à plasma selon l'une des revendications 1 à 4, dans lequel un élément en forme de godet (11) est disposé sur la coque supérieure (4) et un guide (12) réalisé sous forme complémentaire à celui-ci est disposé sur la coque inférieure (3), dans lequel l'élément (11) est guidé lors d'un mouvement de pivotement de la coque supérieure (4).

6. Dispositif à plasma selon l'une des revendications 1 à 5, dans lequel la coque supérieure (4) peut être enlevée de la coque inférieure (3).

7. Dispositif à plasma selon l'une des revendications précédentes, dans lequel une surface de plateau (14) s'étendant dans la direction longitudinale (13) du boîtier (2) comprenant au moins une électrode (15) est agencée sur la coque inférieure (3), par laquelle électrode le plasma peut être appliqué sur la surface à traiter,
dans lequel la surface de plateau (14) est sollicitée par un ressort et fait saillie au-delà du boîtier (2) dans un état de non utilisation du dispositif à plasma (1).

8. Dispositif à plasma selon l'une des revendications précédentes, dans lequel le réservoir d'énergie (6) est une batterie ou un accumulateur.

9. Dispositif à plasma selon l'une des revendications précédentes, dans lequel l'angle d'ouverture α se situe dans un plan longitudinal vertical.

10. Dispositif à plasma selon l'une des revendications précédentes, dans lequel une paroi de protection est disposée sur la coque supérieure (4), qui s'écarte en direction de la coque inférieure (3).
